(19) Europäisches Patentamt
European Patent Office
Office européen des brevets

(11) EP 4 356 935 A1

(12) # EUROPEAN PATENT APPLICATION
published in accordance with Art. 153(4) EPC

(43) Date of publication:
**24.04.2024 Bulletin 2024/17**

(21) Application number: **22825303.5**

(22) Date of filing: **15.06.2022**

(51) International Patent Classification (IPC):
***A61L 27/20*** *(2006.01)* ***A61L 27/52*** *(2006.01)*
***C08B 37/08*** *(2006.01)* ***C08L 5/08*** *(2006.01)*
***C08K 5/1515*** *(2006.01)*

(52) Cooperative Patent Classification (CPC):
**A61L 27/20; A61L 27/52; C08B 37/003; C08K 5/1515; C08L 5/08**

(86) International application number:
**PCT/KR2022/008447**

(87) International publication number:
**WO 2022/265380 (22.12.2022 Gazette 2022/51)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **17.06.2021 KR 20210078632**

(71) Applicant: **Medytox Inc.**
**Chungcheongbuk-do 28126 (KR)**

(72) Inventors:
- **LIM, Cheon Soo**
  **Sejong 30092 (KR)**
- **YANG, Jong Cheol**
  **Cheongju-si Chungcheongbuk-do 28164 (KR)**
- **RHEE, Chang Hoon**
  **Cheongju-si Chungcheongbuk-do 28123 (KR)**

(74) Representative: **Brevalex**
**Tour Trinity**
**1 B Place de la Défense**
**92400 Courbevoie (FR)**

(54) **HYALURONIC ACID CROSS-LINKED PRODUCT, AND FILLER COMPOSITION COMPRISING SAME**

(57) The present disclosure relates to a hyaluronic acid cross-linked product and a filler composition including the same, and more specifically, to a hyaluronic acid cross-linked product with high cohesion and adhesion and reduced swelling, and a filler composition including the same.

FIG. 1

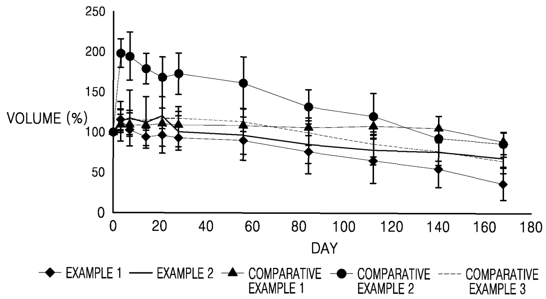


EP 4 356 935 A1

## Description

Technical Field

The present disclosure relates to a hyaluronic acid cross-linked product and a filler composition including the same, and more specifically, to a hyaluronic acid cross-linked product with high cohesion and adhesion and reduced swelling, and a filler composition including the same.

Background Art

Hyaluronic acid (HA) is a linear polysaccharide generally having a high average molecular weight. Hyaluronic acid is a polymer of D-glucuronic acid and N-acetyl-D-glucosamine and has a negative charge. Hyaluronic acid is found primarily in the extracellular matrix and intercellular matrix, but is also present within cells. As such, hyaluronic acid is biocompatible because it is a substance that already exists in the body, and it also has properties that make it easy to prepare cross-linked hyaluronic acid hydrogel by using a cross-linking agent or the like. Therefore, filler products using cross-linked hyaluronic acid have been widely used around the world since Galderma first launched Restylane products in the 1990s.

Hyaluronic acid gel currently used as filler is manufactured by chemically cross-linking natural hyaluronic acid into a network by a cross-linking reaction. While natural hyaluronic acid and certain cross-linked hyaluronic acids absorb water until they are completely soluble in water, cross-linked hyaluronic acid gels typically absorb a certain amount of water until they are saturated, and thus have a limited swelling degree.

Meanwhile, filler products using cross-linked hyaluronic acid gel may be classified as either monophasic or biphasic. Monophasic cross-linked hyaluronic acid gel has a high viscous modulus and a low elastic modulus. Biphasic cross-linked hyaluronic acid gel has a low viscous modulus and a high elastic modulus. Monophasic cross-linked hyaluronic acid gel and biphasic cross-linked hyaluronic acid gel have different properties *in vivo*. For example, in the case of a monophasic cross-linked hyaluronic acid gel with low elastic modulus and high viscous modulus, it has excellent cohesion and is unlikely to escape from the injected area, but because it has the ability to absorb additional water, it has the property of continuously absorbing surrounding moisture even within the body. As a result, monophasic cross-linked hyaluronic acid gel in general has the disadvantage of not maintaining its original form in the initial stage of injection when injected into the body and increasing its volume compared to the initially injected volume. In contrast, biphasic cross-linked hyaluronic acid gel, which has a high elastic modulus but low viscous modulus, may maintain the injected form for a long period of time with a small change in volume, but has the property of being easily dislodged from the injected area or not being evenly distributed in the injected area.

Meanwhile, swelling may occur after hyaluronic acid filler treatment, which refers to a phenomenon in which the hyaluronic acid gel component injected into the body absorbs body fluid from the surrounding tissues of the injection site, causing swelling of the injection site and surrounding areas. Such swelling is one of the common side effects of filler treatment in that the injection site becomes swollen, and this is an undesirable phenomenon in that the viscosity, elasticity, and strength of the hyaluronic acid gel are reduced by the absorbed water, which may reduce the effectiveness of filler injection.

Therefore, there remains a need for a hyaluronic acid filler that maintains suitable viscoelasticity and gel strength when injected into the body while exhibiting little swelling in the body when injected.

Disclosure

Technical Problem

One object of the present disclosure is to provide a hyaluronic acid cross-linked product with a critical strain of 10 % to 100 % and a swelling degree of 1 % to 100 %.

Another object of the present disclosure is to provide a filler composition including the hyaluronic acid cross-linked product.

Technical Solution

One aspect of the present disclosure provides a hyaluronic acid cross-linked product with a critical strain of 10 % to 100 % and a swelling degree of 1 % to 100 %.

In an embodiment, the hyaluronic acid cross-linked product may have an average particle size in the range of 50 μm to 150 μm

In an embodiment, the hyaluronic acid cross-linked product may have an elastic modulus (G') of 400 Pa to

2,000 Pa.

In an embodiment, the hyaluronic acid cross-linked product may have a viscous modulus (G") of 100 Pa to 600 Pa.

In an embodiment, the hyaluronic acid cross-linked product may have a compression of 15 N·s to 100 N·s.

In an embodiment, the hyaluronic acid cross-linked product may have an injection force of 1 N to 50 N.

In an embodiment, the hyaluronic acid cross-linked product may be cross-linked with a cross-linking agent that has a bifunctional epoxy group. The cross-linking agent that has the bifunctional epoxy group may be one or more selected from the group consisting of 1,4-butanediol diglycidyl ether, polyethylene glycol) diglycidyl ether, polypropylene glycol) diglycidyl ether, poly(tetramethylene glycol) diglycidyl ether, polyglycerol polyglycidyl ether, glycerol diglycidyl ether, triethylene diglycidyl ether, trimethylolpropane triglycidyl ether, ethylene diglycidyl ether, neopentyl glycol diglycidyl ether, and 1,6-hexanediol diglycidyl ether.

Another aspect of the present disclosure provide a filler composition including the hyaluronic acid cross-linked product.

In an embodiment, the concentration of the hyaluronic acid cross-linked product in the filler composition may be from 10 mg/mL to 30 mg/mL.

In an embodiment, the filler composition may additionally include no non cross-linked hyaluronic acid.

In an embodiment, the filler composition may further include a local anesthetic.

In an embodiment, the filler composition may be filled in a syringe.

In an embodiment, the filler composition may be used for one or more purposes selected from the group consisting of facial plastic surgery, wrinkle improvement, facial contouring, breast augmentation, breast augmentation, genital enlargement, glans enlargement, urinary incontinence treatment, and arthritis treatment.

Advantageous Effects

The cross-linked hyaluronic acid gel according to the present disclosure is characterized by high cohesion and adhesion, thus remaining intact without being separated from the injection site, while simultaneously reducing swelling after injection.

Description of Drawings

FIG. 1 is a diagram illustrating the volume of injected hyaluronic acid gel according to time elapsed after injection after cross-linked hyaluronic acid samples were injected into a hairless mouse.

Best Mode

The present disclosure generally provide a hyaluronic acid cross-linked product that has a critical strain of 10 % to 100 % and a swelling degree of 1 % to 100 %.

As used herein, the term "hyaluronic acid" refers to a hyaluronan, hyaluronate, or pharmaceutically acceptable salt thereof that has the formula of Formula 1:

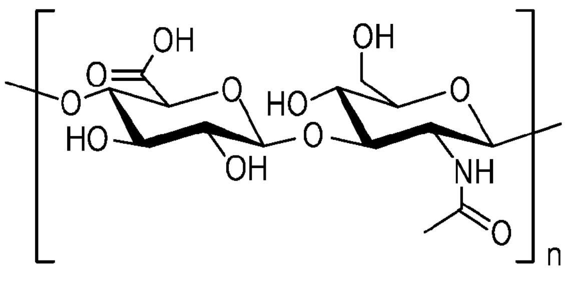

(1)

In Equation 1, n is the number of repeating units. The hyaluronic acid may be of any origin. For example, the hyaluronic acid may be derived from an animal or non-animal source. The hyaluronic acid may also be one derived from bacteria. The bacteria may be from the genus *Streptococcus.* The bacteria of the genus *Streptococcus* may be *Streptococcus equi, S. pyogenes,* or *S. zooepidemicus.* The hyaluronic acid may also be commercially purchased.

The molecular weight of the hyaluronic acid may be 1,000,000 Da to 2,500,000 Da, for example, 1,000,000 Da to 2,000,000 Da, 1,500,000 Da to 2,500,000 Da, or 1,500,000 Da to 2,000,000 Da.

The hyaluronic acid may have an intrinsic viscosity of 1.0 $m^3/kg$ to 5.0 $m^3/kg$, for example, 1.5 $m^3/kg$ to 4.0

$m^3$/kg, 2.5 $m^3$/kg to 4.0 $m^3$/kg, 2.5 $m^3$/kg to 3.5 $m^3$/kg, or 2.5 $m^3$/kg to 3.0 $m^3$/kg.

As used herein, the term "hyaluronic acid cross-linked product" refers to a cross-linked hyaluronic acid, and the terms "hyaluronic acid cross-linked product", "cross-linked hyaluronic acid", and "cross-link hyaluronic acid" are used interchangeably.

As used herein, the term swelling refers to the phenomenon in which a cross-linked hyaluronic acid gel absorbs water and swells.

As used herein, the term swelling degree refers to the ratio of the weight of a fully swollen cross-linked hyaluronic acid gel to its weight before swelling, when the cross-linked hyaluronic acid is immersed in water. The swelling degree may be obtained, for example, according to the equation described in the measurement method section below.

As used herein, critical strain is a measurement index that confirms the strain value required to cause destruction of the internal structure of the substance, including linear viscoelastic properties, when shear strain is applied to the substance. In general, the cross-linked hyaluronic acid gel is consisted of a cross-linked hyaluronic acid gel or a cross-linked hyaluronic acid gel and solution, and various internal interaction forces exist within them, such as a hydrogen bond, covalent bond, van der Waals bond, and physical bond, etc. The internal interaction may be classified as interaction between gel-gel, gel-solution, or solution-solution, in the case of a solution, the fluidity of molecules is higher than that of a gel, and the contact area is smaller than that of a gel, therefore the size of the interaction may be thought of as gel-gel > gel-solution > solution-solution. In the case of a monophasic, where only a gel-gel form exist, the internal structure may be expected to be destroyed at higher strain rates because it has relatively high interactions. On the other hand, in the case of a biphasic, gel and solution exist together, thus the gel-gel interaction is reduced, while the gel-solution and solution-solution interactions are larger, the sum of the interactions in the internal structure is expected to be smaller compared to the monophasic, therefore the internal structure may be expected to be destroyed at lower strain rate.

In addition, as a result of a similar phenomenon, general monophasic filler product may be expected to have strong cohesive properties, while a biphasic filler product may be expected to have relatively weak cohesive properties compared to a monophasic filler. Cohesion property is a property which may be used as a predictive indicator of how well an injectate will hold together *in vivo.* To verify this, adhesion values of biphasic filler product and monophasic filler product were compared, which is a measure of the degree of cohesion, and it was confirmed that the monophasic product exhibited higher adhesion values than the biphasic product.

The hyaluronic acid cross-linked product according to an aspect of the present disclosure exhibit unexpected properties in that they have a high critical strain rate like general monophasic hyaluronic acid fillers, but have low swelling degree. In addition, the hyaluronic acid cross-linked product of the present disclosure has high cohesion and high adhesion similar to a general monophasic filler, so it may not easily escape from the injection site and is characterized by being able to simultaneously maintain the injected form for a long period of time due to high elastic modulus (G').

The hyaluronic acid cross-linked product according to the present disclosure may have (i) a critical strain of 10 % to 100 %, for example 10 % to 50 %, 10 % to 30 %, or 10 % to 40 %, for example 10 % to 50 %, for example 10 % to 30 %, and a swelling degree of 1 % to 100 %, for example 1 % to 90 %, 1 % to 80 %, 1 % to 70 %, 1 % to 60 %, 1 % to 50 %, 10 % to 60 %, 20 % to 60 %, 30 % to 60 %, 40 % to 60 %, 40 % to 50 %, 50 % to 60 %.

The hyaluronic acid cross-linked product may have (ii) an average particle size (um) of 50 μm to 150 μm, for example 60 μm to 150 μm, 70 μm to 150 μm, 80 μm to 150 μm, 90 μm to 150 μm, 60 μm to 140 μm, 60 μm to 130 μm, 60 μm to 120 μm, 60 μm to 100 μm.

The hyaluronic acid cross-linked product of (iii) may have an elastic modulus (G') of 400 Pa to 2,000 Pa, for example 500 Pa to 1,900 Pa, 600 Pa to 1,800 Pa, or 600 Pa to 1,700 Pa.

The hyaluronic acid cross-linked product may have a (iv) viscous modulus (G") of 100 Pa to 600 Pa, for example 120 Pa to 500 Pa, 150 Pa to 450 Pa, or 160 Pa to 350 Pa.

The hyaluronic acid cross-linked product may have (v) a compression of 15 N·s to 100 N·s, for example 20 N·s to 90 N·s, 25 N·s to 80 N·s, or 30 N·s to 75 N·s.

The hyaluronic acid cross-linked product may have an injection force of (vi) of 1 N to 50 N, for example, 3 N to 45 N, 4 N to 40 N, or 7 N to 35 N.

In an embodiment, the hyaluronic acid cross-linked product may fulfill one, two, three, four, or five of the requirements of (ii) to (vi) above while fulfilling the requirement of (i) above.

The hyaluronic acid cross-linked product according to the present disclosure may be prepared by a process including, for example, but not limited to, the following processes:

(a) a process of adding a cross-linking agent to hyaluronic acid dissolved in an alkaline aqueous solution to perform cross-linking;
(b) a process of dialyzing, washing, and/or swelling the cross-linked gel;
(c) a process of pulverizing the gel; and
(d) a process of filling the syringe.

The order of processes (b) to (d) in the above process may be changed or may be repeated two or more times. For example, the pulverizing process may be performed additionally between processes (a) and (b) in addition to process (c), or may be performed only in process (c), or may be performed only in processes (a) and (b) instead of process (c).

Additionally, in addition to processes (a) to (d), other additional processes may be included, for example, adding an anesthetic as described below, and/or performing autoclave. For example, the process of adding an anesthetic may be added between processes (b) and (c) above, or between processes (c) and (d) above. The process of performing autoclave may be added between processes (c) and (d), or after process (d).

In process (a) above, the alkaline aqueous solution may be, but is not limited to, NaOH, KOH, and desirably an aqueous solution of NaOH. If an aqueous solution of NaOH is used, the concentration may be from 0.1 % (w/w) to 2.5 % (w/w), for example from 0.1 % (w/w) to 2.0 % (w/w), or from 0.5 % (w/w) to 1.0 % (w/w).

Furthermore, the concentration of hyaluronic acid dissolved in the alkaline aqueous solution may be 10 % (w/w) to 25 % (w/w), for example 10 % (w/w) to 20 % (w/w), or 10 % (w/w) to 15 % (w/w).

In process (a) above, the cross-linking agent may be one that has a multifunctional group. The cross-linking agent may be one that has a bifunctional epoxy group. The cross-linking agent that has a bifunctional epoxy group may be one or more selected from the group consisting of 1,4-butanediol diglycidyl ether, polyethylene glycol) diglycidyl ether, polypropylene glycol) diglycidyl ether, poly(tetramethylene glycol) diglycidyl ether, polyglycerol polyglycidyl ether, glycerol diglycidyl ether, triethylene diglycidyl ether, trimethylolpropane triglycidyl ether, ethylene diglycidyl ether, neopentyl glycol diglycidyl ether, and 1,6-hexanediol diglycidyl ether.

Furthermore, the cross-linking reaction may be at room temperature or more, for example 20 °C to 40 °C, 25 °C to 40 °C, or 30 °C to 40 °C, and the reaction time may be 24 hours or less, for example 10 hours to 24 hours, 15 hours to 24 hours, or 15 hours to 20 hours.

In process (b) above, dialysis refers to the process of mixing the cross-linked gel with a dialysis solution including a certain buffer solution, for example NaCl solution, KOH solution, PBS solution, etc. in a dialysis membrane or dialysis vessel, dialyzing, washing, and swelling the dialyzed gel. The above series of dialysis, washing and swelling process may be repeated two or more times. Through the series of dialysis, washing, and swelling processes, unreacted cross-linking agent may be removed and/or the pH and/or osmotic pressure of the cross-linked gel may be adjusted.

In process (c) above, the pulverizing may be performed using a pulverizer at 1,000 to 10,000 rpm, for example 2,000 to 9,000 rpm, 3,000 to 8,000 rpm, 4,000 to 7,000 rpm, 5,000 to 9,000 rpm, or 5,000 to 8,000 rpm for at least 60 seconds, for example 60 seconds to 100 seconds, 70 seconds to 120 seconds, 80 seconds to 140 seconds, 90 seconds to 160 seconds, 100 seconds to 180 seconds, 120 seconds to 240 seconds.

Another aspect provides a filler composition including a hyaluronic acid cross-linked product as described above.

In the composition, the final concentration of the cross-linked hyaluronic acid gel may be 10 mg/ml or more, 15 mg/ml or more, 18 mg/ml or more, or 19 mg/ml or more. For example, the concentration of the cross-linked hyaluronic acid may be from 10 mg/ml to 30 mg/ml, 12 mg/ml to 28 mg/ml, 14 mg/ml to 26 mg/ml, 16 mg/ml to 24 mg/ml, 16 mg/ml to 20 mg/ml, or about 18 mg/ml.

The composition may additionally include no non cross-linked hyaluronic acid. "Additionally including" non-cross-linked hyaluronic acid refers to artificially adding more non cross-linked hyaluronic acid to the hyaluronic acid cross-linked product, and "not additionally including" non-cross-linked hyaluronic acid refers to not artificially adding more non cross-linked hyaluronic acid to the hyaluronic acid cross-linked product.

The composition may further include an anesthetic. The anesthetic may be a local anesthetic. The anesthetic may be one or more selected from the group consisting of ambucaine, amolanone, amylocaine, benoxinate, benzocaine, betoxycaine, biphenamine, bupivacaine, butacaine, butamben, butanilicaine, butethamine, butoxycaine, carticaine, chloroprocaine, cocaethylene, cocaine, cyclomethycaine, dibucaine, dimethysoquin, dimethocaine, diperodon, dycyclonine, ecgonidine, ecgonine, ethyl chloride, etidocaine, beta-eucaine, euprocin, fenalcomine, formocaine, hexylcaine, hydroxytetracaine, isobutyl p-aminobenzoate, leucinocaine mesylate, levoxadrol, lidocaine, mepivacaine, meprylcaine, metabutoxycaine, methyl chloride, myrtecaine, naepaine, octacaine, orthocaine, oxethazaine, parethoxycaine, phenacaine, phenol, piperocaine, piridocaine, polidocanol, pramoxine, prilocaine, procaine, propanocaine, proparacaine, propipocaine, propoxycaine, psuedococaine, pyrrocaine, ropivacaine, salicyl alcohol, tetracaine, tolycaine, trimecaine, zolamine, and salts thereof, but is not limited thereto.

The composition may not contain a pharmaceutically active substance selected from the group consisting of a protein, glycosaminoglycan other than hyaluronic acid, and hydroxypropyl methyl cellulose.

The composition may be filled in a syringe.

The composition may be used for tissue repair in a subject.

In the composition, the subject may be a mammal. The mammal may be a human, dog, cat, cow, pig, rat, or sheep.

The composition may be used for one or more purposes selected from the group consisting of facial plastic surgery, wrinkle improvement, facial contouring, breast plastic surgery, breast augmentation, genital enlargement, glans enlargement, urinary incontinence treatment, and arthritis treatment.

The composition may further include a pharmaceutically acceptable carrier, excipient, and diluent. The carrier

may include, for example, water, or a buffer. The buffer may be such that the pH of the solution changes very little with the addition of the components of the composition. The composition may be an aqueous liquid composition. The composition may be an aqueous buffered composition. The pH of the aqueous buffered composition may be in the physiological pH range, for example, between about 6.0 and 8.0. The pH may be adjusted by adding a suitable acid or base, such as HCl, $Na_2CO_3$, or NaOH. In an embodiment, the aqueous buffered composition may include phosphate buffered saline (PBS). In another embodiment, the aqueous buffered composition may include tris(hydroxymethyl)aminomethane (Tris). In some embodiments, additional solutes, such as sodium chloride, calcium chloride, and potassium chloride, may be added to adjust osmolarity and ionic concentration.

The composition may be sterilized.

The composition may be included in a container. The container may be a syringe. The composition may be previously filled into a syringe before use. The composition may be administered with a pre-filled syringe.

Another aspect provides an apparatus including the composition described above. The apparatus may be a pre-filled syringe. The apparatus may be sterilized.

Another aspect provides a kit including the pre-filled syringe. The kit may include an instruction manual including information on administering the composition.

Another aspect provides a method of charging a tissue of a subject, including the process of administering a therapeutically effective amount of the composition to the subject. The method may be for augmenting, repairing or strengthening the tissues of a subject or for filling a body cavity. In this perspective, volume augmentation may be a long-lasting increase in volume caused by a component forming the filler composition. The component forming the filler composition may be those that do not undergo rapid diffusion. In the method above, "composition" and "subject" are as described above. The administration may be administering within the skin, such as the dermis, or within joint cavities. In the method above, the administration may be administering by a syringe, for example a pre-filled syringe, into the skin, for example the dermis. In the method above, the administration may be administering 0.1 to 50 ml, 0.5 to 30 ml, 0.5 to 20 ml, 0.5 to 15 ml, or 0.8 to 12 ml per dose. In the method above, the administration may be administering the composition once per period of at least 3 months, once per period of at least 4 months, once per period of at least 5 months, once per period of at least 6 months, once per period of at least 12 months, or once per period of at least 18 months.

Hereinafter, the present disclosure will be described in more detail through embodiments. However, these embodiments are for illustrative purposes only and the scope of the present disclosure is not limited to these embodiments.

Mode for Invention

## Example

### Measurement method

#### (1) Critical strain measurement method

The critical strain was measured by strain-sweep test. Specifically, a DHR-2 rheometer instrument (TA instruments) was run. The strain-sweep test was calibrated by setting the instrument temperature to 25 °C and mounting a 25 mm diameter geometry. An appropriate amount of sample was loaded centered between the upper and lower Peltier plate geometries of the instrument. The appropriate amount of samples were over-loaded in a sufficient amount to ensure that there was no shortage, so that even if the residue outside the lower area of the geometry was cut off, it did not stain the sides and top of the geometry. After lowering the geometry to the set gap, the sample was checked to ensure that it was well filled under the geometry, and any residual sample outside the geometry was removed by cutting off. The geometry was then used to apply strain to the sample, ranging from 0.1 % to 1000 %, and the elastic modulus value measured at each strain was measured. Among the measured values from the rheometer, the strain % and elastic modulus (Storage Modulus, Pa) values were taken and the strain % value was converted to log (strain (%)) using the built-in Transform function in the program using Graphpad Prism (Prism 8, version 8.4. 3(686)) from Graphpad Software, and in order to easily check the minimum strain % from the obtained value, a smooth graph of the change in elastic modulus (Storage Modulus, Pa) with respect to the log (strain (%)) value was calculated using the Smooth function built into the program, then using the Transform function, log (strain (%)) was converted to strain % , which was the value before taking the log (strain (%)) with the Transform function. From the data obtained by the above procedure, the amount of change in the elastic modulus (Storage Modulus, Pa) value according to the strain % may be checked, and the strain % of the minimum elastic modulus value is defined as the critical strain.

#### (2) Swelling degree measurement method

Hyaluronic acid gel was added to a conical tube, weighed, and the initial weight was recorded. The hyaluronic

acid gel was swollen by adding 5.0 mL of PBS solution (pH 7.4) to the conical tube and vortexing for 1 minute to mix homogeneously, then sealing it completely and incubating it at room temperature for 24 hours. Then, 200 μL of Alcian Blue Solution was added to the swollen hyaluronic acid gel in the conical tube, vortexed for 1 minute, and incubated at room temperature for 30 minutes to stain the hyaluronic acid gel. This was centrifuged at 14000 g at 4°C for 10 minutes to precipitate the hyaluronic acid gel, and the supernatant was carefully removed with a pipette. Then, to remove the dye from the hyaluronic acid gel, 5.0 mL of PBS solution (pH 7.4) was added, vortexed for 1 minute, and centrifuged again at 14000 g at 4°C for 10 minutes. The supernatant was carefully removed with a pipette, and the PBS addition and centrifugation procedure was additionally performed two more times. The swollen hyaluronic acid gel thus obtained was weighed and the swelling degree was calculated using the formula below:

$$\text{Swelling degree (\%)} = \frac{\text{Weight of swollen hyaluronic acid gel} - \text{Weight of hyaluronic acid gel before swelling}}{\text{Weight of hyaluronic acid gel before swelling}} \times 100$$

(3) Average particle size measurement method

The average particle size measurement was performed using the Particle Size Analyzer S3500 from Microtrac. Average particle size measurement was performed by adding a sample to a solvent and was performed using laser diffraction analysis. The solvent was distilled water. Before performing the measurement, the sample inlet was washed with plenty of distilled water, then the refractive index of the distilled water and the sample was input as 1.33 and 1.37, respectively, and the sample type was set to transparent and irregularly shaped particles. Before measurement, the sample inlet of the instrument was filled with distilled water, and then the sample was put into a tube, etc., added with excess distilled water to dilute it, and a vortex, etc. was used to disperse the sample sufficiently before putting it into the instrument for measurement. Among the measurement results, the average particle size (D50) was used as data.

(4) Viscoelasticity measurement method

A DHR-2 rheometer instrument (TA instruments) was run. The viscoelasticity test was calibrated by setting the instrument temperature to 25°C and mounting a 40 mm diameter geometry. An appropriate amount of sample was loaded centered between the upper and lower Peltier plate geometries of the instrument. The sample was over-loaded in sufficient amount to ensure that there was no shortage, and an appropriate amount was loaded to avoid staining the sides and top of the geometry. After lowering the geometry to the set gap, the sample was checked to ensure that it was filled below the geometry, and any residual sample outside the geometry was removed by cutting off. Afterwards, shear strain was periodically applied to the sample according to a specific frequency at a constant geometry strain rate, and among the resulting modulus values, the Storage Modulus and Loss Modulus values at a frequency of about 0.1 Hz were defined as elasticity and viscosity, respectively.

(5) Adhesion measurement method

The adhesion was measured as follows. A DHR-2 rheometer instrument (TA instruments) was run. The adhesion test was calibrated by setting the instrument temperature to 25°C and mounting a 40 mm diameter geometry. An appropriate amount of sample was loaded centered between the upper and lower Peltier plate geometries of the instrument. The sample was over-loaded in sufficient amount to ensure that there was no shortage, and an appropriate amount was loaded to avoid staining the sides and top of the geometry. After lowering the geometry to the set gap, the sample was checked to ensure that it was well filled below the geometry, and any residual sample outside the geometry was removed by cutting off. Afterwards, the geometry gap was set to 1000 μm in height, and the force applied to the geometry was measured when the lower Peltier plate was pulled in the vertical axis direction at a constant speed of 100.0 μm/s for 180 seconds. Due to the adhesion of the sample between the geometry and the Peltier plate, the largest force was measured at the moment the initial sample falls, and this force was defined as adhesion.

(6) Compression measurement method

A DHR-2 rheometer instrument (TA instruments) was run. The compression test was calibrated by setting the instrument temperature to 25°C and mounting a 25 mm diameter geometry. A 1 mL sample was loaded centered between the upper and lower Peltier plate geometries of the instrument. After lowering the geometry to the set gap, the geometry was rotated at a slow speed to ensure that the sample may be in the exact center of the geometry. Afterwards, the force applied to the geometry was measured by lowering the geometry from 2500 μm to 900 μm at a constant speed of 13.33 μm/s. Compression was defined as the value corresponding to the value of the area under the graph, which is the integral

value of the graph, when a graph was drawn with the force measured from the beginning to the end of the test as the Y-axis and the moving time as the X-axis.

(7) Injection force measurement method

The injection force was measured using Mecmesin's Multitest 2.5-i Universal Testing Machine. The load cell to measure the force applied to the machine was used by mounting an appropriate load cell with a tolerance value higher than the injection force measurement range. A syringe containing a sample was placed under the machine load cell, and a needle was attached to the syringe. A push bar with a flat end was placed on the syringe to ensure that a constant force may be applied when the load cell applied force. After adjusting the distance until the load cell portion was just about touching the end of the push bar, the load cell was used to push the push bar portion at a rate of 12 mm/min to measure the force applied. At the beginning and end of the measurement, the pressure value generated when the sample flows into the needle is lower than the pressure applied when injecting the syringe. In addition, when the injection of the injection material is almost completed, there may be cases where force is continued to be applied even though there is no sample, and in this case, the applied force is unrelated to the injection force of the sample. Therefore, the injection force was defined as the average of the injection force values measured in the middle portion of the syringe filling, excluding the initial injection force and the final injection force values, and excluding the values corresponding to the part where the sample flows into the needle and the part where the injection is almost completed.

**Examples 1 and 2: Preparation of cross-linked hyaluronic acid**

A hyaluronic acid cross-linked product that has a critical strain of 10% to 100% and a swelling degree of 1% to 100% was prepared as follows.

First, a 1 % (w/w) NaOH solution was prepared. To the prepared 1 % NaOH solution, 5 g of sodium hyaluronate (IV 2.2 to 3.0) was mixed to 13.00 % (w/w) and stirred to dissolve sufficiently. Here, IV refers to intrinsic viscosity. To the above solution, 0.630 g of butanediol diglycidyl ether (BODE) (Sigma-Aldrich) was added and further stirred to mix well, and then taken out and subjected to a cross-linking reaction at 40 °C for 16 hours to 20 hours to prepare a cross-linked hyaluronic acid gel.

Next, the obtained cross-linked gel was placed in a dialysis membrane and sealed, and dialysis was performed using 1 mol/kg NaCl aqueous solution and 1x PBS aqueous solution as a dialysis solution to remove unreacted cross-linking agent. After completing dialysis, content correction was performed using 1x PBS so that the final concentration of sodium hyaluronate removed was 18 mg/mL, taking into account the loss rate from the weight of sodium hyaluronate initially added. For lidocaine hydrochloride hydrate, the content was corrected to 3 mg/mL. As a result, a PBS solution containing 18 mg/mL of cross-linked hyaluronic acid and 3 mg/mL of lidocaine was prepared. The prepared cross-linked hyaluronic acid-containing solution was pulverized at 8000 rpm for 180 seconds using a blender (Retsch GM-200). Thereafter, 1 ml of the pulverized cross-linked hyaluronic acid-containing solution was filled into a glass syringe, and autoclave was performed. The substance obtained by this procedure was designated as Example 1.

For Example 2, the cross-linked hyaluronic acid-containing solution was prepared in the same manner as Example 1, except that the cross-linked hyaluronic acid-containing solution was ground using a blender (Retsch GM-200) at 5000 rpm for 75 seconds.

**Comparative Example 1**

Comparative Example 1 was prepared in the same manner as Example 1, except that the cross-linked hyaluronic acid-containing solution was ground using a blender (Retsch GM-200) at 2000 rpm for 180 seconds.

**Comparative Example 2 and Comparative Example 3:**

Juvederm Voluma Lidocaine from Allergan, a monophasic cross-linked hyaluronic acid, was used as Comparative Example 2, and Restylane Lyft Lidocaine from Galderma, a biphasic cross-linked hyaluronic acid, was used as Comparative Example 3.

**Example 1: Measurement of physical properties of cross-linked hyaluronic acid gel**

The physical properties of the cross-linked hyaluronic acid gel were measured by the methods described in measurement methods (1) to (7) in the above Example, and the results are shown in Table 1 below.

[Table 1]

| Name | Critical strain (%) | Swelling degree (%) | Average particle size (μm) | G' (Pa) | Adhesion (N) | Compression (Ns) | Injection force (N) | Cross-linking degree (%) |
|---|---|---|---|---|---|---|---|---|
| Example 1 | 26.01 | 56.30 | 66.0 | 611 | 6.27 | 50.09 | 16 (27G 1/2") | 4.1 |
| Example 2 | 14.69 | 49.40 | 113.3 | 622 | 6.07 | 49.12 | 18 (27G 1/2") | 4.13 |
| Comparative Example 1 | 4.49 | 67.10 | 261.6 | 824 | 4.82 | 47.08 | 11 (27G 1/2") | 3.89 |
| Comparative Example 2 | 54.96 | 126.1 | 660.7 | 227 | 5.11 | 45.60 | 8 (27G) | 2.08 |
| Comparative Example 3 | 6.53 | N/A (Not measurable) | 809.0 | 743 | 2.42 | 22.18 | 19 (29G) | 0.16 |

As shown in Table 1, it may be seen that the swelling degree of Example 1 and Example 2 was significantly reduced compared to Comparative Example 2 (Juvederm Voluma L, Allergan), which is a general monophasic cross-linked hyaluronic acid filler. In the case of Comparative Example 3 (Restylane Lyft L, Galderma), which is a general biphasic cross-linked hyaluronic acid filler, the swelling degree could not be measured due to a problem in which the cross-linked hyaluronic acid gel and the solution were not separated when measuring the swelling degree.

Considering the results of the physical properties in Table 1 above, it may be seen that Example 1 and Example 2 have compression and adhesion similar to those of general monophasic fillers, making them less likely to dislodge from the injection site, and at the same time, have low swelling degree, which significantly reduces the swelling phenomenon when injected into the body. In addition, the fact that it has a high elastic modulus (G') shows that the injected form may be maintained for a long period of time.

**Example 2: Measuring lifting and volumizing effects of cross-linked hyaluronic acid when applied to animals: lifting and volumizing test**

Fifty-two 6-week-old female (20±3 g) hairless mice (SKH1-hr) were obtained from OrientBio (Republic of Korea) and acclimatized for approximately 1 week or more. The acclimated mice were anesthetized by intraperitoneal injection of a mixture of ketamine (100 mg/kg) and rumfun (10 mg/kg), and then 0.1 mL each of Example 1 to Example 2 and Comparative Example 1 to Comparative Example 3 were injected subcutaneously at the dorsal region site using a glass syringe. After injection, the body weight of the subject was measured according to the designated date. For the injection site, the height of the injection site, the maximum length of the injection, and the volume were measured using Primos 5.8E (Canfield Scientific Inc, NJ, USA). In addition, the injection site was visually observed and photographs were taken.

Figure 1 shows the volume change observed up to 42 weeks after intradermal injection of hyaluronic acid gel. As shown in Figure 1, in the case of Comparative Example 1, which is a general monophasic cross-linked hyaluronic acid gel, the initial volume of injection increases significantly (about 100% increase compared to the initial injection volume) and the volume decreases significantly over time, whereas Example 1 and Example 2 showed little or no change in volume compared to the initial injection (approximately 10% increase compared to the initial injection volume). Furthermore, it may be seen that Example 1 and Example 2 show relatively little volume loss over the course of 24 weeks. In summary, Example 1 and Example 2 confirmed that the swelling phenomenon was small in the early stages after injection, which may allow the original volume to be maintained upon injection, and that the volume change up to 24 weeks after injection was small, demonstrating excellent in vivo persistence and biocompatibility.

## Claims

1. A hyaluronic acid cross-linked product that has a critical strain of 10 % to 100 % and a swelling degree of 1 % to 100 %.

2. The hyaluronic acid cross-linked product of claim 1, having an average particle size in a range of 50 μm to 150 μm.

3. The hyaluronic acid cross-linked product of claim 1, having an elastic modulus (G') of 400 Pa to 2,000 Pa.

4. The hyaluronic acid cross-linked product of claim 1, having a viscous modulus (G") of 100 Pa to 600 Pa.

5. The hyaluronic acid cross-linked product of claim 1, having compression of 15 N·s to 100 N·s.

6. The hyaluronic acid cross-linked product of claim 1, having an injection force of 1 N to 50 N.

7. The hyaluronic acid cross-linked product of claim 1, cross-linked with a cross-linking agent that has a bifunctional epoxy group.

8. The hyaluronic acid cross-linked product of claim 7, wherein the cross-linking agent that has a bifunctional epoxy group is one or more selected from the group consisting of 1,4-butanediol diglycidyl ether, polyethylene glycol) diglycidyl ether, polypropylene glycol) diglycidyl ether, poly(tetramethylene glycol) diglycidyl ether, polyglycerol polyglycidyl ether, glycerol diglycidyl ether, triethylene diglycidyl ether, trimethylolpropane triglycidyl ether, ethylene diglycidyl ether, neopentyl glycol diglycidyl ether, and 1,6-hexanediol diglycidyl ether.

9. A filler composition comprising the hyaluronic acid cross-linked product of claim 1.

10. The composition of claim 9, wherein a concentration of the hyaluronic acid cross-linked product is 10 mg/mL to 30 mg/mL.

11. The composition of claim 9, wherein the composition does not additionally comprise non cross-linked hyaluronic acid.

12. The composition of claim 9, further comprising a local anesthetic.

13. The composition of claim 9, wherein the composition is filled in a syringe.

14. The composition of claim 9, wherein the composition is for use for one or more purposes selected from the group consisting of facial plastic surgery, wrinkle improvement, facial contouring, breast plastic surgery, breast augmentation, genital enlargement, glans enlargement, urinary incontinence treatment, and arthritis treatment.

FIG. 1

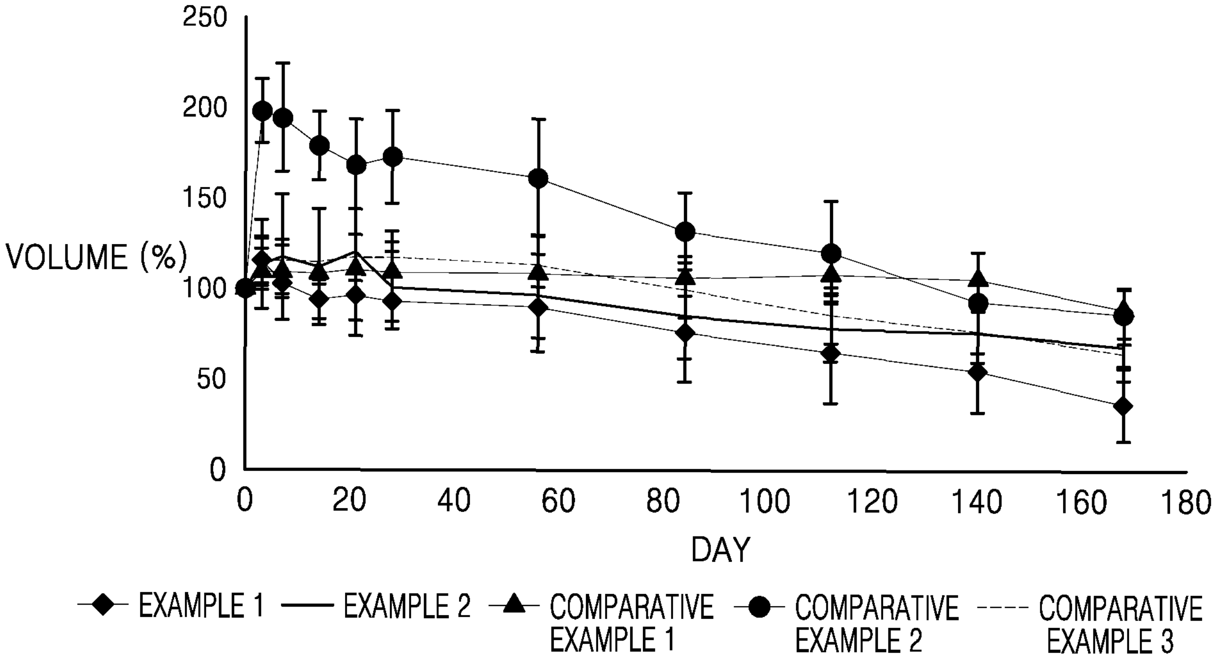
250
200
150
VOLUME (%)
100
50
0
0
20
40
60
80
100
120
140
160
180
DAY
EXAMPLE 1
EXAMPLE 2
COMPARATIVE EXAMPLE 1
COMPARATIVE EXAMPLE 2
COMPARATIVE EXAMPLE 3

**INTERNATIONAL SEARCH REPORT**

International application No. **PCT/KR2022/008447**

**A. CLASSIFICATION OF SUBJECT MATTER**

**A61L 27/20**(2006.01)i; **A61L 27/52**(2006.01)i; **C08B 37/08**(2006.01)i; **C08L 5/08**(2006.01)i; **C08K 5/1515**(2006.01)i

According to International Patent Classification (IPC) or to both national classification and IPC

**B. FIELDS SEARCHED**

Minimum documentation searched (classification system followed by classification symbols)

A61L 27/20(2006.01); A61K 31/07(2006.01); A61K 31/728(2006.01); A61K 9/50(2006.01); C08F 283/00(2006.01); C08J 3/075(2006.01); C08J 3/24(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 히알루론산 가교체(cross-linked hyaluronic acid), 충전제 조성물(filler composition), 임계 변형률(critical strain), 팽윤도(swelling degree), 가교제(cross-linking agent)

**C. DOCUMENTS CONSIDERED TO BE RELEVANT**

| Category* | Citation of document, with indication, where appropriate, of the relevant passages | Relevant to claim No. |
|---|---|---|
| X<br>Y | STOROZHYLOVA, N. et al. An in situ hyaluronic acid-fibrin hydrogel containing drug-loaded nanocapsules for intra-articular treatment of inflammatory joint diseases. Regenerative Engineering and Translational Medicine. 2020, vol. 6, pp. 201-216.<br>See Abstract; Syringeability Studies and Rheological behaviour of Hydrogels; and Figures 2, 3 and 5. | 1-6,9-10,12-14<br>7-8,11 |
| Y | US 2018-0223053 A1 (GALDERMA SA) 09 August 2018 (2018-08-09)<br>See paragraphs [0012]-[0124]; and figures 1-4. | 7-8,11 |
| A | SEONG, Y. et al. Hyaluronic acid-based hybrid hydrogel microspheres with enhanced structural stability and high injectability. ACS Omega. 2019, vol. 4, pp. 13834-13844.<br>See 2. RESULTS AND DISCUSSION; 3. CONCLUSIONS and 4. EXPERIMENTAL SECTION; and Figures 1-7. | 1-14 |
| A | KR 10-2236812 B1 (FOUNDATION FOR RESEARCH AND BUSINESS, SEOUL NATIONAL UNIVERSITY OF SCIENCE AND TECHNOLOGY) 06 April 2021 (2021-04-06)<br>See entire document. | 1-14 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **02 September 2022** | **22 September 2022** |
| Name and mailing address of the ISA/KR<br>**Korean Intellectual Property Office**<br>**Government Complex-Daejeon Building 4, 189 Cheongsa-ro, Seo-gu, Daejeon 35208**<br>Facsimile No. **+82-42-481-8578** | Authorized officer<br><br>Telephone No. |

Form PCT/ISA/210 (second sheet) (July 2019)

**INTERNATIONAL SEARCH REPORT**

International application No.
**PCT/KR2022/008447**

**C. DOCUMENTS CONSIDERED TO BE RELEVANT**

| Category* | Citation of document, with indication, where appropriate, of the relevant passages | Relevant to claim No. |
|---|---|---|
| A | KR 10-2016-0020509 A (GALDERMA S.A.) 23 February 2016 (2016-02-23)<br>See entire document. | 1-14 |
| A | KR 10-2008-0073419 A (HANSON BIOTECH CO., LTD.) 11 August 2008 (2008-08-11)<br>See entire document. | 1-14 |

Form PCT/ISA/210 (second sheet) (July 2019)

**INTERNATIONAL SEARCH REPORT**
**Information on patent family members**

International application No.
**PCT/KR2022/008447**

| Patent document cited in search report | Publication date (day/month/year) | Patent family member(s) | Publication date (day/month/year) |
|---|---|---|---|
| US 2018-0223053 A1 | 09 August 2018 | EP 3328351 A1 | 06 June 2018 |
| | | US 11021580 B2 | 01 June 2021 |
| | | US 2021-0155763 A1 | 27 May 2021 |
| | | WO 2017-016917 A1 | 02 February 2017 |
| KR 10-2236812 B1 | 06 April 2021 | KR 10-2021-0006195 A | 18 January 2021 |
| KR 10-2016-0020509 A | 23 February 2016 | AU 2014-280303 A1 | 18 December 2014 |
| | | CA 2914765 A1 | 18 December 2014 |
| | | CN 105451744 A | 30 March 2016 |
| | | JP 2016-524644 A | 18 August 2016 |
| | | US 2016-0129134 A1 | 12 May 2016 |
| | | WO 2014-198683 A2 | 18 December 2014 |
| | | WO 2014-198683 A3 | 19 February 2015 |
| KR 10-2008-0073419 A | 11 August 2008 | None | |

Form PCT/ISA/210 (patent family annex) (July 2019)